# EUROPEAN PATENT SPECIFICATION

(11) **EP 1 094 756 B1**
(45) Date of publication and mention of the grant of the patent: **14.01.2009**
(21) Application number: 99933865.0
(22) Date of filing: 08.07.1999
(51) Int. Cl.: A61B 17/04

(54) **Devices for securing sutures and ligatures without knots**
Vorrichtungen zur Sicherung von Nahtmaterial und Abbindungen ohne Knoten
Dispositifs permettant de realiser des sutures et des ligatures sans noeuds

(30) Priority: 08.07.1998 US 92072 P; 08.07.1998 US 92074 P
(43) Date of publication of application: 02.05.2001
(73) Proprietor: Axya Medical Inc., Beverly, MA 01915 (US)
(72) Inventor: FENTON, Paul, V., Marblehead, MA 01945 (US)
(74) Representative: Hill, Justin John
(86) International application number: PCT/US1999/015627
(87) International publication number: WO 2000/002489

(56) References cited:
- US-A- 3 513 848
- US-A- 5 383 883
- US-A- 5 425 489
- US-A- 5 586 938
- US-A- 5 618 311

## Description

### Field of the Invention

The present invention relates generally to devices for joining sutures together without knots, and for securing living tissue structures together without sutures.

### Background of the Invention

In minimally invasive surgical procedures that use elongated instruments and videoscopic viewing of the surgery site, there is a significant elevation in the difficulty of knot tying and wound approximating. Traditional methods of wound closure routinely involve the use of individual hand- knotted sutures. The suture strands are directed through portions of tissue to be joined and formed into a single stitch, which is then knotted. However, due to the location of the area being sutured, the delicate nature of anatomical features, and the stiffness of the suture used, it can be difficult to tie uniform stitches to close the wound that do not unravel or tie off (or ligate) a vessel. Non-uniform stitches (i.e., stitches of varying tension) or varied bite size (depth into the tissue) can cause uneven healing, localized trauma, infection, and patient discomfort. To reduce the discomfort and aid healing, it is desirable to secure sutures uniformly and close to a wound. Due to the stiffness of some sutures, knotting the sutures can be difficult, particularly when the tissue to be sutured is deep within the body. Typical knots may be relatively large and elevated above the tissue being sutured, which can increase patient discomfort.

It is also desirable in many surgical procedures where sutures are used to reduce the size of the knot bundle and the amount of foreign material in the body.

The knot bundle can become an irritant and retard the healing process and cause discomfort or pain for the patient. The knot bundle can also be a source of infection.

Methods known in the art to overcome these problems include various suture securing devices such as buttons, and methods of fusing synthetic sutures. Although buttons can produce sutures with even tension and without the concomitant dexterity of knot tying, their elevated location above the wound or within the body cavity can cause irritation and discomfort. Furthermore, there is a risk of button migration, since they are discrete objects in the body.

Suture fusion techniques, whereby synthetic polymer suture strands are melted together by the application of heat or other energy to the sutures, are known in the art. Examples of devices to perform such suture fusion are disclosed in US Patent No. 5,417,700, assigned to the assignee of this application. However, some polymeric sutures are not amenable to this process. For example, braided or multi-filament sutures may not completely fuse since spaces between the individual strands may interfere with the heat or energy transfer needed for fusion to occur. As a result, the sutures may be incompletely fused, and the resulting joint may fail.

It would be advantageous to provide suture and tissue joining devices which are fusible to and/or around sutures and other structures, including living tissue, so as to avoid the need for suture knots.

US Patent No. 5,383,883 (D2) discloses a method for ultrasonically applying a surgical device.

### Summary of the Invention

According to one aspect of the invention, there is provided a device for securing one or more elongated members or the same elongated member looped upon itself. The device is a flexible, fusible collar that is disposed about a central region which extends about a central axis. The collar extends circumferentially between two end portions and is biasable into a nominally closed position so that the end portions of the collar overlap. In this form the collar is adapted to encircle a portion of the elongated members. The overlapping portions of the collar are adapted to fuse to each other around the elongated members upon application of sufficient energy to the overlapping portions of the collar.

In one embodiment, the elongated members are surgical sutures which are made of a fusible material. The sutures are adapted to fuse at least to each other and possibly also to the collar in a knotless weld upon application of sufficient energy to the collar.

In another embodiment, the sutures are made of non-fusible, or minimally fusible material and only the collar is providing the welded interface. In another embodiment, the sutures are made of braided material that is marginally fusible. In another embodiment, the elongated members are a band of material that encircles a bundle of other structures, such as blood vessels.

The collar is preferably made of a thermoplastic polymeric material. The elongated members can be surgical sutures which are also made of a fusible, thermoplastic polymeric material. In another embodiment, the elongated members can be living tissue structures. The energy may be generated from a variety of sources known in the art, such as for example, thermal energy, optical energy, radiofrequency energy, current sources or more preferably, ultrasonic energy.

In a preferred embodiment, the collar includes one or more energy directors extending from a surface of the collar. The energy directors define fusion regions of the sutures and the collar and are adapted to focus energy applied to the collar to the fusion regions so that the sutures and collar fuse together preferentially at the fusion regions. The energy directors can be located along the inner surface of the collar, at the opposing surfaces of the overlap region, or both.

The inside surface of the collar may also be smooth, or have protrusions, grooves or other texturing to aid in securing the encircled structures.

According to another aspect of the invention, there is provided a kit for securing one or more elongated living tissue structures. The kit includes a fusible collar as described above, and a fusing tool which compresses the collar around the tissue structures so that the tissue structures are securely retained within the collar and portions of the collar overlap. The fusing tool applies energy to the collar to fuse the overlapped portions of the collar together around the tissue structures. The fusing tool includes generally an energy source, a welding head, an end effector, and general electronics, switches, control devices and the like for supplying weld energy and activating the end effector.

In one embodiment of the fusing tool, the energy source is ultrasonic energy. The fusing tool preferably includes an ultrasonic transducer, an ultrasonic welding horn, and an end effector. The end effector may include one or more jaw members adapted for selective deployment around at least a portion of the collar to form an ultrasonic welding anvil. In various embodiments, the end effector can be a pair of opposable jaws which move with respect to each other, or a pair of jaws which are resiliently biased toward each other.

In alternate embodiments, energy for bonding is supplied by thermal energy (e.g., heat), optical energy (e.g., laser generated), electrical energy (e.g., radio frequency, RF), or current sources (e.g., resistive heating).

According to another aspect of the invention, there is provided a kit for joining a plurality of surgical sutures together without a knot. The kit provides a fusible collar as described above, and a fusing tool as described above, for compressing the collar around the sutures so that the sutures are securely retained within the collar. The fusing tool applies energy to the collar to fuse the overlapping portions of the collar to each other and may also fuse portions of the collar to portions of the sutures.

These and other objects and advantages of the invention will in part be obvious and will in part appear hereinafter. The invention accordingly comprises the apparatus possessing the construction, combination of elements and arrangement of parts which are exemplified in the following detailed disclosure, the scope of which will be indicated in the claims.

### Brief Description of the Drawings

For a fuller understanding of the nature and objects of the present invention, reference should be made to the following detailed description taken in connection with the accompanying drawings, in which:
Figure 1 is an oblique view of a preferred embodiment of the collar of the invention;
Figure 2A is a cross-sectional view of a fused collar around a pair of suture strands, taken along section lines B-B of FIG. 1;
Figure 2B is a cross sectional view of a fused collar around a tubular structure such as a vessel, taken along section lines B-B of Figure 1;
Figure 3A is an oblique view of another preferred embodiment of the collar;
Figure 3B is an oblique view of another preferred embodiment of the collar displaying a modified inner surface;
Figure 4 is an oblique view of yet another embodiment of the collar;
Figure 5 is an oblique view of an end effector for a fusing tool;
Figure 6 is a detail view of the end effector of Figure 5 deployed around a collar encircling sutures to be fused together;
Figure 7 is an oblique view of an alternate embodiment of an end effector for a fusing tool;
Figure 8A is a detail view of the end effector of Figure 7 positioned around a collar encircling sutures to be fused together;
Figure 8B shows the cross section of the collar release mechanism for the embodiment shown in Figure 7.
Figures 9A-9F are simplified diagrams of a method of securing sutures in a collar according to one aspect of the invention; shows a wound through which a suture strand has been passed;
Figure 10 is an oblique view of the collar ligating a vessel; and
Figures 11A and 11B are oblique views of another embodiment of the invention.

Like features in the figures are labeled with like numerals.

### Description of the Preferred Embodiments

The present invention provides, in one aspect, a device, and kit, for securing sutures or anatomical features in human or animal tissue that eliminates the need for tying knots in sutures. The invention is practiced with the aid of a tool that joins a fusible banding device or collar to itself around the sutures or around living tissue structures, such as ligaments, vessels and ducts. Alternatively, the collar can be fused to itself around a number of sutures, which can also be fused to one another.
The devices and methods of the invention secure a fusible collar to sutures or living tissue structures close to a wound site so that the sutures can be made taut and secured without coming undone and without the bulk and inconvenience associated with knots and knotting processes, which are difficult, time-consuming and cumbersome in confined spaces. Furthermore, the joining process insures that braided and multi-filament sutures will fuse adequately and remain secure. Various methods of fusing or joining sutures or other elongated structures together joining by the application of energy to the fusible collar are well known in the art and can be employed to secure the band and sutures. Examples of such techniques include, but are not limited to, thermal energy (e.g., heat), optical energy (e.g. laser), electrical (e.g., radiofrequency RF), current sources (e.g., resistive heating), and preferably, ultrasonic energy.

Application of energy to the fusible collar may be carefully controlled, as detailed more fully below, to ensure localized melting and joining of the collar without causing trauma to underlying and nearby tissue structures.

In a preferred form of the invention, the collar is formed as a split, or open, ring-like structure which is made of a biocompatible, flexible material that is adapted to allow sutures, and/or other anatomical structures (vessels, ducts, ligaments and the like) to be encircled by it. The collar may be resilient or substantially resilient so that after cessation of a deforming force, it returns to substantially its original shape. The collar can be made in various geometries and may have one or more energy directors extending from a surface of the collar to focus energy propagating through the collar at particular areas, or fusion regions, on the collar. Such fusion regions may be regions of contact between the collar and sutures contained therein, so that fusion occurs preferentially at those regions during compression of the collar and application of energy thereto.

Throughout the specification, it will be understood that the collar can be used to encircle living tissue structures and cinch or bundle them together, wherein the collar is then fused to itself at an overlap region to secure the bundled structures together. Similarly, the collar can encircle one living tissue structure, such as a blood vessel or duct, then fuse to itself in a manner that restricts the flow of fluid through the structure (ligate). Alternatively, the collar can be used to encircle and secure a plurality of fusible surgical sutures, which can themselves be melted and fused together, and optionally to the collar, upon application of energy to the collar.

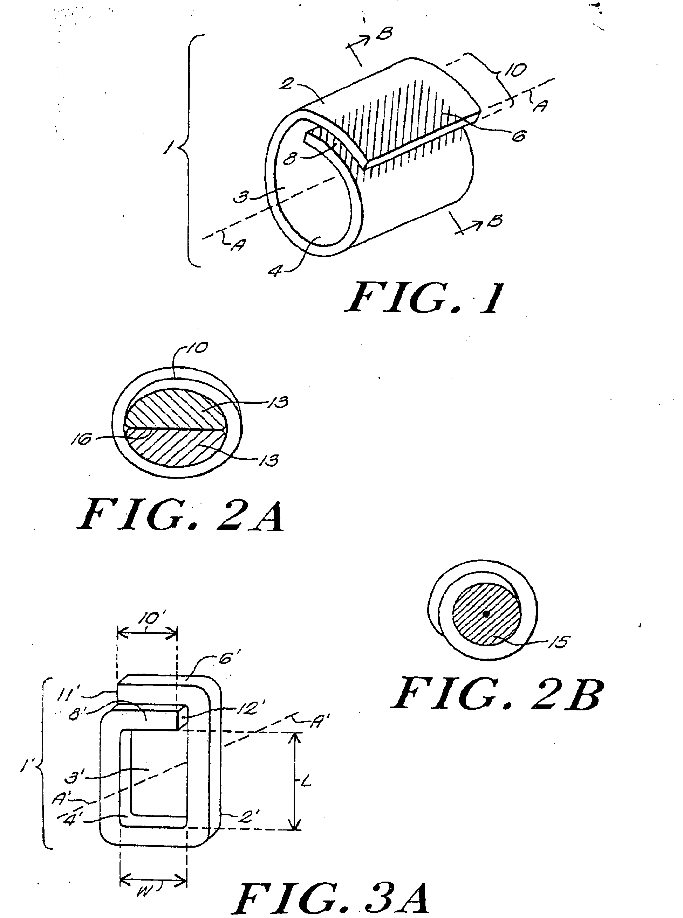

Figure 1 shows one embodiment of the collar **1** which is formed substantially as a "c" shape and which is resiliently biased into a nominally closed "o"-shaped position so as to encircle a generally cylindrical central region **3** extending along central axis **A** for retaining sutures or living tissue structures. End portion **6** extends circumferentially and overlaps end portion **8** at overlap area **10** to form a tubular structure.

Central region **3** of the tubular structure should be large enough to permit the sutures or tissue structures to be secured therein by the collar so that the its end portions **6, 8** overlap. The inner surface **4** of the collar can be smooth, or it can be grooved, have protrusions, or be otherwise textured in some way, to create a roughened surface area to enhance contact between the collar and the structure(s) therein. This latter form is particularly useful when securing a collar to a tissue such as a ligament.

Figure 2A shows the collar after it has been compressed so that end portions **6, 8** overlap. Following the compression, collar **1** is fused around a pair of sutures **13.** Preferably, collar **1** is compressed around the sutures **13,** so that the sutures are pressed against each other to the point of deformation, thereby increasing their mutual contact surface areas. Figure 2B shows the collar compressed around and ligating an anatomical structure, such as a blood vessel **15.**

Depending on the selection of materials for the collar and the sutures, fusion can occur in selected regions, i.e., in the overlap region **10** of the collar, between the collar and the sutures at energy directors, discussed more fully below, and at interface regions **16** between the sutures themselves.

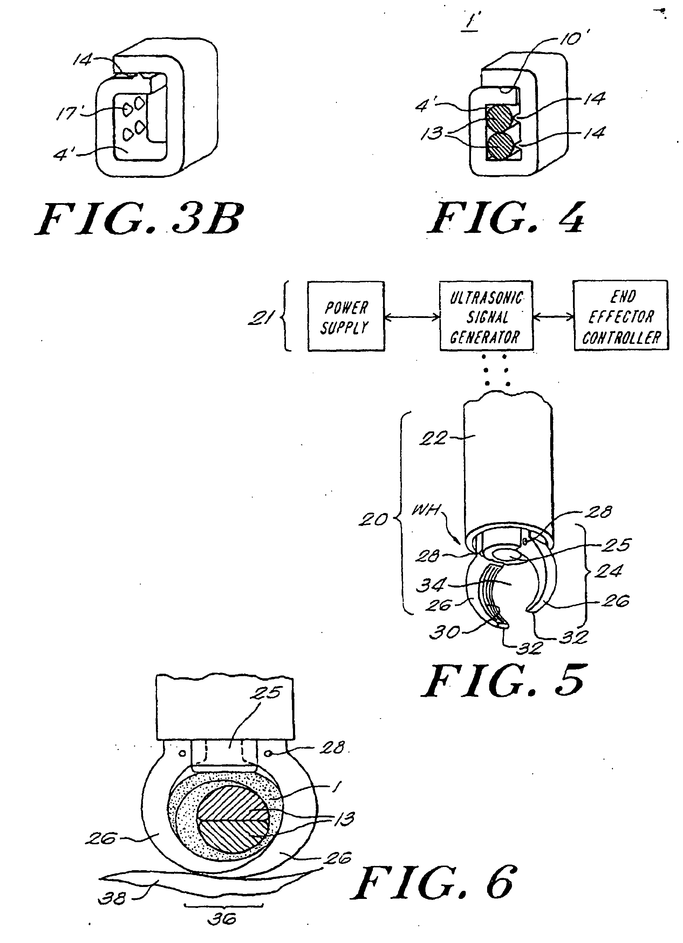

Figures 3A and 3B show the details of alternate embodiments of the collar **1'.** In Figure 3A, collar **1',** like collar **1,** is fashioned from a flexible, biocompatible material. Collar **1**' is also formed in substantially a "c" shape, albeit with a squared shape forming central region **3'** about a central axis **A'** for retaining the structures. In this embodiment, central region **3'** may have length (**L**) and width (**W**) forming substantially a rectangular shape. In a preferred embodiment, **W** is approximately equal to or slightly larger than the diameter of one structure to be secured therein; length **L** is approximately equal to or greater than the diameter of two structures to be secured therein. This dimensioning of the collar maintains the sutures or ligaments therein in a preferred alignment with the welding horn during the fusing process, thereby providing for optimal transmission of the bonding energy to the contact areas. Other dimensions for the collar in this example are considered to be within the scope of the invention.

To form the tubular fused structure, end portion **6'** overlaps end portion **8'** at overlap area **10'.** Longitudinally running edge **12'** on the end portion **8'** may be in contact with inner surface **4'** or spaced apart from inner surface **4'** to give the collar an open, or "c", shape. Inner surface **4'** may be smooth, or it can be grooved, or have protrusions or be otherwise textured to enhance engagement of the sutures and collar for efficient fusing or welding. Figure 3B shows a collar **1'** which is similar to the collar in Figure 3A, except that it has cone-shaped protrusions **17'** extending inwardly from inner surface **4'.** Other shapes for protrusions may be used.

To permit attached tissue structures to be encircled by collar **1',** end portions **6'** and **8'** may be eased apart in opposite directions so as to admit and encircle the structures. Alternately, sutures or non-attached structures may be threaded through the central region **3'** of the collar.

An alternate embodiment of the collar is shown in Figure 4. This embodiment contains all the details found in the embodiment shown in Figure 3 with the addition of one or more energy directors **14** located on a contact surface, illustrated here as inner surface **4'.** The energy directors **14** focus the applied energy at particular points, or fusion regions, for example, at the junction between the sutures and inner surface of the collar or on the opposing surfaces of the end portions at the overlap area. The energy directors insure that welds are formed preferentially at these locations.

The collar is preferably made of a biocompatible material which is sufficiently flexible to allow for moderate deformation of the collar to allow sutures or other anatomical structures to be placed within the collar. The collar may be substantially resilient so that after cessation of a deforming force, it returns substantially to its original shape around the sutures or structures therein. In one form, the material is resilient so as to retain the structures within the collar and create contact surfaces or fusion regions for the welds. Any type of material that meets these requirements can be used.

The collar material is preferably also capable of being fused or joined together upon the application of energy, such as thermal energy (heat), optical energy (laser generated), electrical energy (radio frequency, RF), current sources (resistive heating) or, preferably, ultrasonic energy, to the collar. Preferred materials are synthetic polymers capable of being repeatedly softened or melted with the application of heat or pressure (commonly known as thermoplastics). Thermosetting plastics and other heat-fusible materials may also be suitable for use as a collar under certain conditions.

The collar can be made by methods known in the art, such as, but not limited to, machining, injection molding, extrusion, thermoforming and the like.

If desired, a collar made of one material, and sutures made of a different material having a different melting temperature, can be employed together so as to further direct the melting and fusing upon application of energy to the collar. Higher melting point materials may be preferred for the collar, particularly if braided or multi-filament sutures are used, as the bonding energy can fuse underlying fibers as well as the sutures themselves. The energy required to melt the material using the various processes and the time required for the molten material to resolidify are well known in the art.

The suture material can be of any type customarily used for sutures such as silk, but preferred materials are polymers such as PTFE, and especially preferred are thermoplastic materials, such as polyamide (nylon), polypropylene, polyester, polyglycolic acid (PGA), polyglyconate, and polydioxanone. The sutures can be either substantially monofilamentous, multiple stranded, twisted, braided, or otherwise interlinked material. The suture filament can have any cross-sectional shape, for example, substantially circular, elliptical or rectangular.

The choice of materials for the sutures and the collar and the geometry of the collar determines which materials fuse, and where fusing occurs. For example, if a collar and sutures are made from materials with largely differing melting temperatures (for example, collars are thermoset polymers or non polymer material, and the suture strands are thermoplastic polymers), the bonds can occur at the suture to suture interface, and within the individual fibers that make up the suture strand (in the case of a multi filament or braided material), but little or no fusing of sutures to the collar occurs. Conversely, when the collar is made of a thermoplastic material, and the suture is a thermoset polymer, non polymer or when an anatomical feature is within the collar, fusing of the collar to itself at the overlap areas occurs. This could be beneficial for retaining ligaments, vessels or ducts, while allowing the collar to move relative to the ligaments, vessels or ducts retained therein, or for ligating a vessel or duct. In a most preferred embodiment, the collar material and the sutures are both made of a material that melts at similar temperatures (e.g. both are thermoplastic polymers). This allow for bonds to occur at all or any of the contact interfaces described.

The fusing tool used to compress and fuse the collar generally is shown in FIGS. 5-8. The fusing tool **20** includes a shaft **22,** welding head **WH,** and end effector **24** adapted to cradle and compress the collar around sutures or structures to be joined. The shaft also connects the welding head and end effector to various control devices **21** and electronics for receiving electrical power and converting it to weld energy, and for moving the end effector. Welding head **WH** transmits the weld energy to the collar.

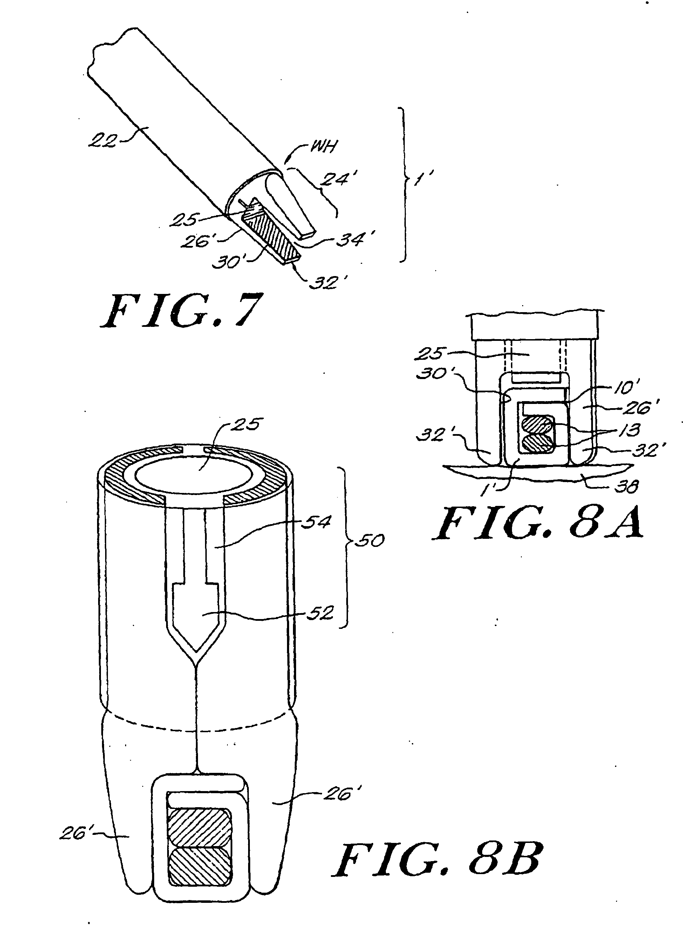

In the ultrasonic embodiment, the welding head **WH** is adapted to intimately contact the surface of the collar when the collar is positioned within the end effector. End effector **24** is mounted to the shaft and surrounds the welding horn. In one embodiment, shown in FIG. 7, the end effector comprises a plurality of securing prongs, which also function as a welding anvil, and can be variously configured for use with collars of differing geometries and can be interchanged on the shaft.

Figure 5 shows the fusing tool **20** as it appears in preparation to receive a collar. The fusing tool **20** includes a delivery shaft **22** with end effector **24** attached to the distal end of the delivery shaft at joints **28,** which can be pins, hinges or the like. End effector **24** is formed by a pair of curved jaws **26** which move on joints **28** relative to each other to open and close to receive, hold and release the collar **1**. Inner surfaces **30** of jaws **26** can be smooth, grooved or otherwise textured to enhance engagement between the jaws and the collar. Welding horn **25** is situated between the joints on delivery shaft **22.**

Figure 6 shows a fusing tool **20** with a collar 1 positioned near tissue **38** for fusing the collar around and to a pair of sutures **13.** Jaw ends **32** abut or overlap to form welding anvil **36.** Deployment of the jaws around the collar compresses the collar **1** around sutures **13** to maintain tension on the sutures and increase the contact surface area of the suture and collar, allowing for a larger total fused area. To fuse the collar to itself and to the sutures, bonding energy is transmitted from the welding horn **25** to anvil **36,** through the collar and the sutures within the collar. The fused collar and encased sutures are released from the jaws when jaws **26** are retracted.

Figure 7 shows an alternate configuration of the fusing tool **20'**. In this embodiment, welding horn **25** is mounted at the distal end of delivery shaft **22** and surrounded by the end effector **24'**, which is formed from a plurality of resiliently mounted rigid prongs **26'** extending from the shaft **22**. The prongs can expand slightly to accept collar **1'**, and then contract to hold the collar securely and apply moderate compression to the collar and the structures within the collar.

Prongs **26'** define an aperture **34'** for holding collar **1'** during positioning, compression and fusing of the collar and structures therein. Prongs **26'** are spaced apart by sufficient distance to allow collar **1'** to fit snugly yet releasably therebetween. Inner surfaces **30'** of prongs **26'** can be smooth, grooved or otherwise textured to enhance contact between the suture collar **1'** and the prongs. Flattened or rounded ends **32'** on the prongs allow for the tip to abut tissue **38** when the sutures are pulled tight through the collar, such as at a wound site, thereby minimizing any gaps in the tissue to be joined and maintaining a desired tension on the sutures. In one embodiment, the prongs can also function as an anvil. In other embodiments, underlying bone, tissue, anatomical features, or other materials temporarily or permanently placed under the end effector may also act as an anvil. The latter design may be preferable if it is necessary or otherwise advantageous to secure the collar as closely as possible to tissue so as to permit minimal gapping between the joined sutures and the collar.

Figure 8A shows a collar and two sutures in a position to be fused. Fusing tool **20'** engages collar **1'** as shown. A preferred orientation is shown in Figure 8; however, other collar orientations are within the scope of the invention. For example, the collar can be rotated so that the weld overlap area **10'** is parallel to the inner surface **30'** of the prongs **26'**.

Prongs **26'** can flex slightly to expand and accommodate the collar within aperture **34'** and hold the collar snugly, yet releasably within the aperture. This moderate compression of the collar within the aperture can also further compress the sutures within the collar, increasing the contact interfaces between the sutures and the collar. This is especially preferred when braided sutures are used, as the compressive force on braided sutures reduces the volume of air spaces between the braid filaments. As the fusing tool is energized, fusing energy is transferred to the collar and sutures. Fusing may occur, for example, at the overlap area **10',** between the inner surface **30'** of the collar and the sutures **13,** and between the suture strands themselves. Since the collar is frictionally held within the aperture **34'** by the prongs **26'**, it can be easily removed from the opening after the weld is completed.

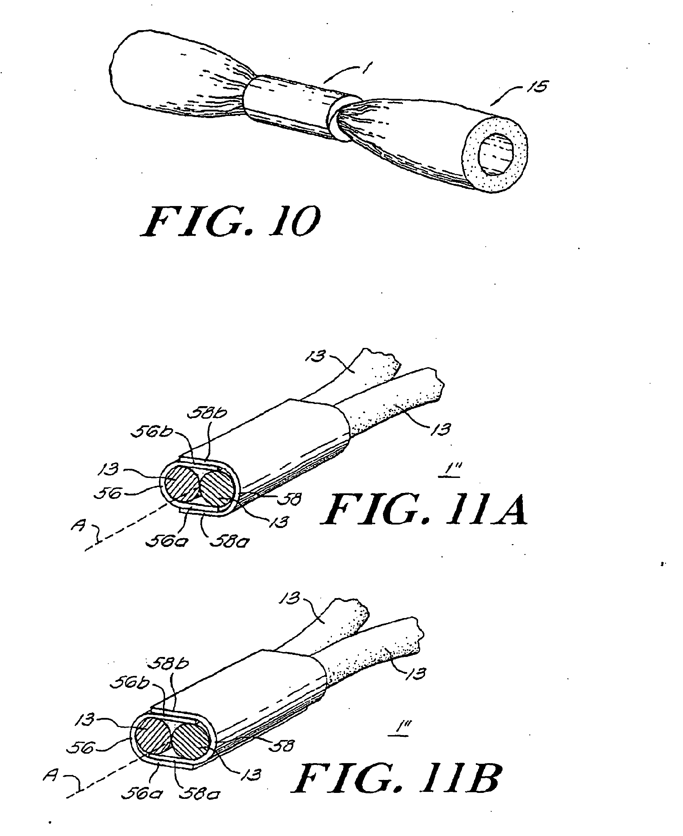

In an alternate embodiment, the tool includes a collar ejection mechanism **50,** an example of which is shown in Figure 10. In this example, the injection mechanism includes one or more wedge shaped members **52,** located within a recess or channel **54** in shaft, exterior to the welding horn. The release mechanism is moved downwardly toward the collar, separating prongs **26'.** This separation causes a space to open between the prongs, thereby releasing the bonded collar from the assembly. Other release mechanism are also envisioned, for example, a mechanism whereby the ultrasonic horn **25** is moved downwardly into aperture **34'** toward the prong ends and pushes the collar free from the prongs.

In another embodiment of the invention, the fusing tool end effector and collar comprise a kit. The kit may include various end effectors that are interchangeable on the tool shaft for accommodating different collar shapes and sizes.

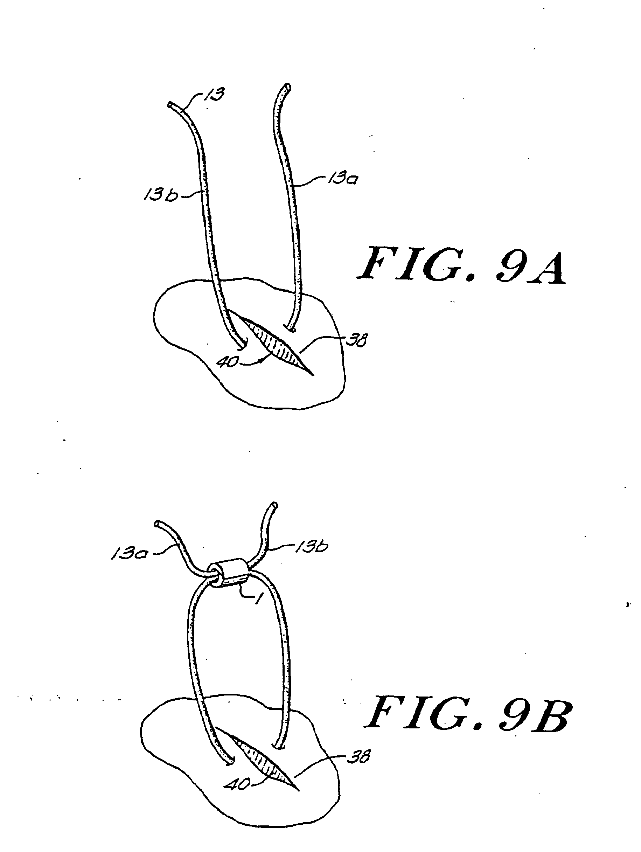

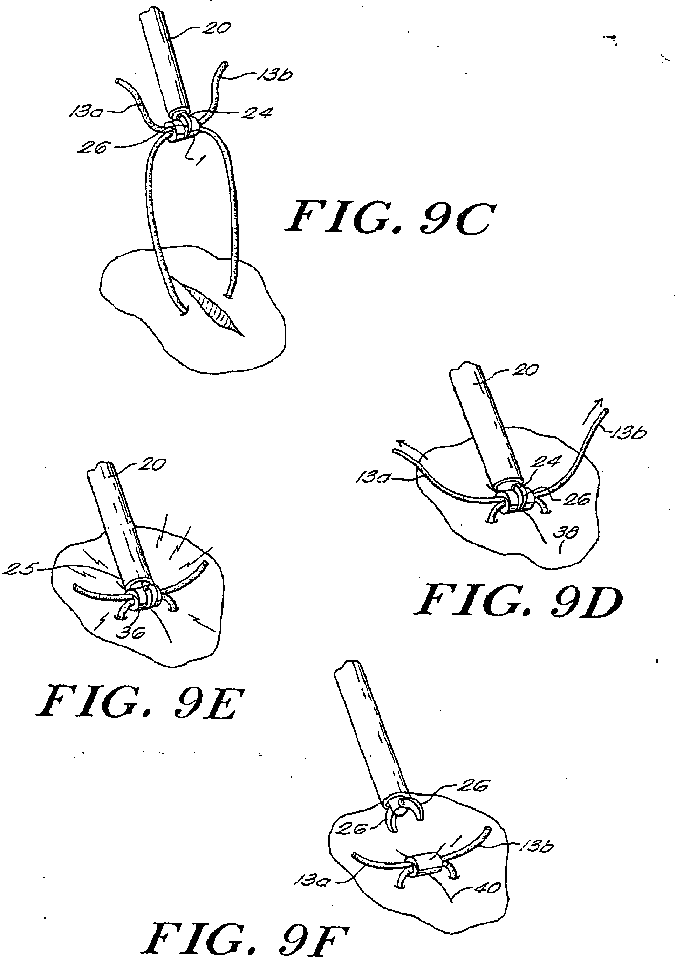

A process for securing suture strands within a fusible collar is demonstrated in Figures 9A-9F using the collar 1 shown in Figure 1 and the fusing tool **20** of Figure 5. Similar procedures are used with the other embodiments. In Figure 9A, a suture **13** has been passed through opening or fissure **40** in tissue **38.** In Figure 9B the suture strands **13 a,b** are threaded in opposite directions through collar **1** prior to fusing. In Figure 9C, collar **1** is engaged by the jaws **26** of the fusing tool **20.** In Figure 9D, the strands **13a,b** are pulled taut in opposite directions as indicated by the arrows, thereby applying tension on opposite edges of the wound fissure **40** to close it. As shown in FIG. 9E, to secure the collar in place for welding and maintain the tension on the suture, the jaws **26** compress the collar **1** around the suture strands. Ultrasonic or thermal energy is transferred from the welding horn **25** to anvil **36** and collar **1,** creating localized heat, which melts the collar **1** sufficiently to cause plastic flow and fusing of the collar to itself and to the sutures **13** encased therein. The fused collar remains within the jaws for a short period of time to allow the fused material to re-solidify. Figure 9F shows jaws **26** retracted from the collar **1** and the fused collar-suture structure in place securing wound **40.** Suture strands **13a,b** can be trimmed close to the collar without loosening the fused structure.

This method for closing a wound can be employed for other surgical procedures and anatomical structures. For example, the process for ligating a vessel or duct is similar to the one described except that the suture is threaded under or around the vessel or duct rather than through tissue, and the suture is secured with an appropriate collar. Alternatively, a collar can be placed around the vessel or duct, compressed, and fused to itself. Figure 10 shows collar 1 fused to itself around a crimped vessel **15,** completing the ligation.

Figures 11A and 11B show alternate forms of the invention (collar assembly 1") which are generally similar to the embodiment of Figure 1, but instead of a single piece structure which extends circumferentially from end portions 6 and 8, the collar assembly 1" is made of two U-shaped cross section elements 56 and 58. Element 56 extends between end portions 56a and 56b, and element 58 extends between end portions 58a and 58b. Elements 56 and 58, when connected as shown in Figures 11A and 11B, form a functionally similar structure to the collar 1 of Figure 1. In use, for example, the collar assembly 1" is disposed about two sutures 13. Then, a fusing tool, for example, of the type shown in Figure 5, is positioned about collar assembly 1", and energy is applied to effect fusion of the mutually adjacent overlapping end portions 56a, 58a, and 56b, 58b. While elements 56 and 58 are shown to have smooth, continuous cross sections from end portion to end portion, these elements, in alternate forms, may have piecewise continuous (e.g., polygonal shaped) cross sections.

Although preferred and other embodiments of the invention are described herein, further embodiments may be perceived by those skilled in the art without departing from the scope of the invention as defined by the following claims.

## Claims

1. A device for securing one or more elongated members comprising:
a collar (1) disposed about a central region (3), said central region extending along a central axis (A) for containing at least a portion of said elongated members aligned with said central axis, wherein said collar extends circumferentially between two end portions and is made of a flexible, fusible material and wherein said collar is biasable into a nominally closed position so that said end portions (6, 8) of said collar overlap, and wherein said overlapping portions of said collar are adapted to fuse to each other around said central region (3) upon application of sufficient energy to at least one of said overlapping portions of said collar (1).

2. A device for securing one or more elongated members comprising:
a collar (1') assembly disposable about a central region (3'), said central region extending along a central axis for containing at least a portion of said elongated members aligned with said central axis (A'), wherein said collar assembly includes two U-shaped cross-sectioned elements, each extending between a first end portion (6') and a second end portion (8') wherein said U-shaped elements are adapted to interfit to extend circumferentially about said central region (3'), with said first end portions mutually adjacent and said second end portions mutually adjacent, being made of a flexible, fusible material, and wherein said collar assembly, when interfit, is biasable into a nominally closed position so that said first end portions of said U-shaped elements overlap, and said second end portions of said U-shaped elements overlap, and wherein said overlapping end portions of said U-shaped elements are adapted to fuse to each other around said central region (3') upon application of sufficient energy to at least one of said overlapping portions of said collar assembly.

3. The device according to claim 1, wherein the elongated members comprise surgical sutures (13) made of a fusible material, wherein the sutures are adapted to fuse to at least one of the collar (1) and to one another in a knotless weld upon application of sufficient energy to the collar.

4. The device according to claim 2, wherein the elongated members comprise surgical sutures (13) made of a fusible material, wherein the sutures are adapted to fuse to at least one of the collar (1') assembly and to one another in a knotless weld upon application of sufficient energy to the collar assembly.

5. The device according to claim 3, wherein the collar includes one or more energy directors extending from an inwardly facing surface of the collar into the central region, wherein the energy directors define fusion regions of the sutures and the collar and are adapted to focus energy applied to the collar to the fusion regions so that one or more of the elongated members and collar are fusible together at the fusion regions.

6. The device according to claim 4, wherein one or both U-shaped elements include one or more energy directors extending from an inwardly facing surface of said one or both U-shaped elements into the central region, wherein the energy directors define fusion regions of the sutures and the collar assembly and are adapted to focus energy applied to the collar assembly to the fusion regions so that one or more of the elongated members and collar assembly are fusible together at the fusion regions.

7. The device according to any of claims 1, or 3 wherein the collar includes one or more energy directors located on one or more opposing surfaces of the end portions in the overlap area, wherein the energy directors define fusion regions for the end portions within overlapped area and are adapted to focus energy applied to the collar to the fusion regions so that the end portions are fusible together.

8. The device according to claim 2, or 4 wherein one or both U-shaped elements include one or more energy directors located on one or more opposing surfaces of the end portions thereof in the overlap area, wherein the energy directors define fusion regions for the end portions within overlapped areas and are adapted to focus energy applied to the collar assembly to the fusion regions so that the end portions are fusible together.

9. The device according to any of the preceding claims wherein the elongated members comprise a band of material adapted for securing a plurality of anatomical structures together.

10. The device according to any of claims 1, 3, 5, or 7 wherein the collar includes one or more protrusions extending from an inwardly facing surface of the collar into the central region and are adapted to secure the elongated members within the central region.

11. The device according to any of claims 2, 4, 6, or 8 wherein the collar assembly includes one or more protrusions extending from an inwardly facing surface of one or both u-shaped elements into the central region when interfit and are adapted to secure the elongated members within the central region.

12. The device according to claims 1 or 2 wherein the elongated members comprise living tissue structures.

13. The device according to any of the preceding claims wherein the energy applied to the collar or collar assembly is ultrasonic energy.

14. The device according to any of the preceding claims wherein the energy applied to the collar or collar assembly is selected from the group consisting of thermal energy, optical energy, electrical energy and current sources.

15. The device according to any of the preceding claims wherein the collar assembly is made of a thermoplastic polymeric material.

16. A kit for securing one or more elongated living tissue structures, the kit comprising:
a device according to claim 1, wherein the one or more elongated members are living tissue structures; and
a fusing tool (20) for compressing the collar around the tissue structures so that the tissue structures are securely retained within the central region, and for applying energy to the collar to fuse the overlapped portions together, wherein the fusing tool includes an energy source, a welding head (WH) for effecting the fusion, and an end effector (24) for supporting the collar during compression and fusion.

17. The kit according to claim 16 wherein the energy source is an ultrasonic transducer, and the fusing tool further includes a welding horn extending between the ultrasonic transducer and the welding head for transmitting ultrasonic energy from the transducer to the welding head.

18. A kit according to claim 16 or 17 wherein the end effector includes one or more jaw members adapted for selective deployment around at least a portion of the collar to form an ultrasonic welding anvil.

19. A kit for joining a plurality of surgical sutures together without a knot, the kit comprising:
a device according to claim 1, wherein the one or more elongated members are surgical sutures; and
a fusing tool (20) for compressing the collar around the surgical sutures so that the surgical sutures are securely retained within the central region, and for applying energy to the collar to fuse the overlapped portions together, wherein the fusing tool includes an energy source, a welding head (WH) for effecting the fusion, and an end effector (24) for supporting the collar during compression and fusion.

20. A kit according to claim 19 wherein the energy source is an ultrasonic transducer, and the fusing tool further includes a welding horn extending between the ultrasonic transducer and the welding head for transmitting ultrasonic energy from the transducer to the welding head, and wherein the end effector includes one or more jaw members adapted for selective deployment around at least a portion of the collar to form an ultrasonic welding anvil.

21. A kit according to any of the claims 16,17,19 or 20 , wherein the end effector includes a pair of opposed jaws which are moveable with respect to each other.

22. A kit according to claim 18, wherein the jaw members are opposed and moveable with respect to each other.

23. A kit according to any of the claims 16, 17, 19 or 20 , wherein the end effector includes a pair of jaws which are resiliently biased toward each other.

24. A kit according to claim 18, wherein the jaw members are resiliently biased toward each other.

## Patentansprüche

1. Vorrichtung zum Sichern bzw. Befestigen eines oder mehrerer länglicher Glieder, umfassend:
einen Bund (1), der um einen zentralen Bereich (3) herum angeordnet ist, wobei sich der zentrale Bereich entlang einer zentralen Achse (A) erstreckt, um zumindest einen Abschnitt der länglichen Glieder zu enthalten, der bzw. die mit der zentralen Achse ausgerichtet bzw. in eine Linie gebracht sind, wobei sich der Bund umfangsmäßig zwischen zwei Endabschnitten erstreckt und aus flexiblem, schmelzbarem Material besteht, und wobei der Bund in eine nominal geschlossene Position vorspannbar ist, so dass die Endabschnitte (6, 8) des Bunds überlappen, und wobei die überlappenden Abschnitte des Bunds angepasst sind, miteinander um den zentralen Bereich (3) herum zu verschmelzen, wenn ausreichend Energie an zumindest einen der überlappenden Abschnitte des Bunds (1) angelegt wird.

2. Vorrichtung zum Sichern bzw. Befestigen eines oder mehrerer länglicher Glieder, umfassend:
eine Bund (1')-Anordnung, die um einen zentralen Bereich (3') herum anordnbar ist, wobei sich der zentrale Bereich entlang einer zentralen Achse erstreckt, um zumindest einen Abschnitt der länglichen Glieder zu enthalten, der bzw. die mit der zentralen Achse (A') ausgerichtet bzw. in eine Linie gebracht sind, wobei die Bundanordnung zwei U-förmige Querschnittselemente enthält, die sich jeweils zwischen einem ersten Endabschnitt (6') und einem zweiten Endabschnitt (8') erstrecken, wobei die U-förmigen Elemente angepasst sind, ineinanderzupassen, um sich umfangsmäßig um den zentralen Bereich (3') herum zu erstrecken, wobei die ersten Endabschnitte aneinander angrenzen bzw. benachbart zueinander sind und die zweiten Endabschnitte aneinander angrenzen bzw. benachbart zueinander sind, bestehend aus einem flexiblen, schmelzbaren Material, und wobei die Bundanordnung, wenn sie ineinander gepasst ist, in eine nominal geschlossene Position vorspannbar ist, so dass die ersten Enidabschnitte der U-förmigen Elemente überlappen und die zweiten Endabschnitte der U-förmigen Elemente überlappen, und wobei die überlappenden Endabschnitte der U-förmigen Elemente angepasst sind, miteinander um den zentralen Bereich (3') herum zu verschmelzen, wenn ausreichend Energie an zumindest einen der überlappenden Abschnitte der Bundanordnung angelegt wird.

3. Vorrichtung nach Anspruch 1, wobei die länglichen Glieder chirurgisches Nahtmaterial bzw. Fäden (13) umfassen, die aus einem schmelzbaren Material bestehen, wobei die Fäden angepasst sind, an zumindest eines von Bund (1) und an- bzw. miteinander in einer knotenfreien Schweißnaht zu verschmelzen, wenn ausreichend Energie an den Bund angelegt wird.

4. Vorrichtung nach Anspruch 2, wobei die länglichen Glieder chirurgisches Nahtmaterial bzw. Fäden (13) umfassen, die aus einem schmelzbaren Material bestehen, wobei die Fäden angepasst sind, an zumindest eines von Bund (1')-Anordnung und an- bzw. miteinander in einer knotenfreien Naht zu verschmelzen, wenn ausreichend Energie an den Bund angelegt wird.

5. Vorrichtung nach Anspruch 3, wobei der Bund einen oder mehrere Energierichter enthält, die sich von einer nach innen gewandten Fläche bzw. Oberfläche des Bunds in den zentralen Bereich hinein erstrecken, wobei die Energierichter Schmelzbereiche der Fäden und des Bunds definieren und angepasst sind, an den Bund angelegte Energie auf die Schmelzbereiche zu fokussieren, so dass eines oder mehrere der länglichen Glieder und der Bund an den Schmelzbereichen miteinander verschmelzbar sind.

6. Vorrichtung nach Anspruch 4, wobei ein oder beide U-förmige Elemente einen oder mehrere Energierichter enthalten, die sich von einer nach innen gewandten Fläche bzw. Oberfläche des einen oder der beiden U-förmigen Elemente in den zentralen Bereich hinein erstrecken, wobei die Energierichter Schmelzbereiche der Fäden und der Bundanordnung definieren und angepasst sind, an die Bundanordnung angelegte Energie auf die Schmelzbereiche zu fokussieren, so dass eines oder mehrere der länglichen Glieder und die Bundanordnung an den Schmelzbereichen miteinander verschmelzbar sind.

7. Vorrichtung nach einem der Ansprüche 1 oder 3, wobei der Bund einen oder mehrere Energierichter enthält, die an einer oder mehreren gegenüberliegenden bzw. entgegengesetzten Flächen bzw. Oberflächen der Endabschnitte in dem Überlappungsbereich angeordnet sind, wobei die Energierichter Schmelzbereiche für die Endabschnitte in Überlappungsbereichen definieren und angepasst sind, an den Bund angelegte Energie auf die Schmelzbereiche zu fokussieren, so dass die Endabschnitte miteinander verschmelzbar sind.

8. Vorrichtung nach Anspruch 2 oder 4, wobei ein oder beide U-förmige Elemente einen oder mehrere Energierichter enthalten, die an einer oder mehreren gegenüberliegenden bzw. entgegengesetzten Flächen bzw. Oberflächen davon in dem Überlappungsbereich angeordnet sind, wobei die Energierichter Schmelzbereiche für die Endabschnitte in Überlappungsbereichen definieren und angepasst sind, an die Bundanordnung angelegte Energie auf die Schmelzbereiche zu fokussieren, so dass die Endabschnitte miteinander verschmelzbar sind.

9. Vorrichtung ach einem der vorhergehenden Ansprüche, wobei die länglichen Glieder ein Materialband bzw. -streifen umfassen, das angepasst ist, eine Mehrzahl anatomischer Strukturen miteinander zu befestigen.

10. Vorrichtung nach einem der Ansprüche 1, 3, 5 oder 7, wobei der Bund einen oder mehrere Vorsprünge enthält, die sich von einer nach innen gewandten Fläche bzw. Oberfläche des Bunds in den zentralen Bereich hinein erstrecken und angepasst sind, die länglichen Glieder in dem zentralen Bereich zu befestigen.

11. Vorrichtung nach einem der Ansprüche 2, 4, 6 oder 8, wobei die Bundanordnung einen oder mehrere Vorsprünge enthält, die sich von einer nach innen gewandten Fläche bzw. Oberfläche von einem oder beiden U-förmigen Elemente in den zentralen Bereich hinein erstrecken, wenn diese ineinander gepasst sind, angepasst sind, die länglichen Glieder in dem zentralen Bereich zu befestigen.

12. Vorrichtung nach Anspruch 1 oder 2, wobei die länglichen Glieder Lebendgewebestrukturen umfassen.

13. Vorrichtung nach einem der vorhergehenden Ansprüche, wobei die an den Bund oder die Bundanordnung angelegte Energie Ultraschallenergie ist.

14. Vorrichtung nach einem der vorhergehenden Ansprüche, wobei die an den Bund oder die Bundanordnung angelegte Energie aus der Gruppe bestehend aus Wärmeenergie, optischer Energie, elektrischer Energie und Stromquellen ausgewählt ist.

15. Vorrichtung nach einem der vorhergehenden Ansprüche, wobei die Bundanordnung aus einem thermoplastischen Polymermaterial besteht.

16. Satz bzw. Kit zum Sichern bzw. Befestigen von einer oder mehreren Lebendgewebestrukturen, wobei der Satz umfasst:
eine Vorrichtung nach Anspruch 1, wobei das eine oder die mehreren länglichen Glieder Lebendgewebestrukturen sind; und
ein Schmelzwerkzeug (20) zum Zusammendrücken des Bunds um die Gewebestrukturen herum, so dass die Gewebestrukturen sicher bzw. fest in dem zentralen Bereich gehalten sind, und zum Anlegen von Energie an den Bund, um die überlappenden Abschnitte miteinander zu verschmelzen, wobei das Schmelzwerkzeug eine Energiequelle, einen Schweißkopf (WH) zum Bewirken des Verschmelzens und einen Endeffektor (24) zum Stützen bzw. Tragen des Bunds während dem Zusammendrücken und Schmelzen enthält.

17. Satz nach Anspruch 16, wobei die Energiequelle ein Ultraschallwandler ist und das Schmelzwerkzeug ferner ein Schweißhorn enthält, das sich zwischen dem Ultraschallwandler und dem Schweißkopf erstreckt, um Ultraschallenergie von dem Wandler an den Schweißkopf zu übertragen.

18. Satz nach Anspruch 16 oder 17, wobei der Endeffektor ein oder mehrere Bakkenglieder enthält, die angepasst sind, selektiv um zumindest einen Abschnitt des Bunds herum eingesetzt zu werden, um einen Ultraschallschweißamboss zu bilden.

19. Satz bzw. Kit zum knotenlosen Verbinden einer Mehrzahl chirurgischer Fäden, wobei der Satz umfasst:
eine Vorrichtung nach Anspruch 1, wobei das eine oder die mehreren Glieder chirurgische Fäden sind; und
ein Schmelzwerkzeug (20) zum Zusammendrücken des Bunds um die chirurgischen Fäden herum, so dass die chirurgischen Fäden sicher bzw. fest in dem zentralen Bereich gehalten sind, und zum Anlegen von Energie an den Bund, um die überlappenden Abschnitte miteinander zu verschmelzen, wobei das Schmelzwerkzeug eine Energiequelle, einen Schweißkopf (WH) zum Bewirken des Verschmelzens und einen Endeffektor (24) zum Stützen bzw. Tragen des Bunds während dem Zusammendrücken und Schmelzen enthält.

20. Satz nach Anspruch 19, wobei die Energiequelle ein Ultraschallwandler ist und das Schmelzwerkzeug ferner ein Schweißhorn enthält, das sich zwischen dem Ultraschallwandler und dem Schweißkopf erstreckt, um Ultraschallenergie von dem Wandler an den Schweißkopf zu übertragen, und wobei der Endeffektor ein oder mehrere Backenglieder enthält, die angepasst sind, selektiv um zumindest einen Abschnitt des Bunds herum eingesetzt zu werden, um einen Ultraschallschweißamboss zu bilden.

21. Satz nach einem der Ansprüche 16, 17, 19 oder 20, wobei der Endeffektor ein Paar entgegengesetzter bzw. gegenüberliegender Backen umfasst, die zueinander beweglich sind.

22. Satz nach Anspruch 18, wobei die Backenglieder gegenüberliegend bzw. entgegengesetzt sind und zueinander beweglich sind.

23. Satz nach einem der Ansprüche 16, 17, 19 oder 20, wobei der Endeffektor ein Paar Backen umfasst, die rückstellfähig zueinander hin vorgespannt sind.

24. Satz nach Anspruch 18, wobei die Backenglieder rückstellfähig zueinander hin vorgespannt sind.

## Revendications

1. Dispositif pour fixer ensemble un ou plusieurs éléments allongés comprenant :
un collier (1) disposé autour d'une région centrale (3), ladite région centrale s'étendant le long d'un axe central (A) pour contenir au moins une partie desdits éléments allongés alignés avec ledit axe central, dans lequel ledit collier s'étend de façon circonférentielle entre deux extrémités et se compose d'un matériau flexible et fusible et dans lequel ledit collier peut être placé en biais dans une position normalement fermée de sorte que lesdites extrémités (6, 8) dudit collier se chevauchent, et dans lequel lesdites parties de chevauchement dudit collier sont adaptées pour fusionner entre elles autour de ladite région centrale (3) suite à l'application d'une énergie suffisante sur au moins une desdites parties de chevauchement dudit collier (1).

2. Dispositif pour fixer ensemble un ou plusieurs éléments allongés comprenant :
un ensemble formant collier (1') pouvant être disposé autour d'une région centrale (3'), ladite région centrale s'étendant le long d'un axe central pour contenir au moins une partie desdits éléments allongés alignés avec ledit axe central (A'), dans lequel ledit ensemble formant collier comprend deux éléments en coupe en forme de U, chacun s'étendant entre une première extrémité (6') et seconde extrémité (8') dans lequel lesdits éléments en forme de U sont adaptés pour s'inter-ajuster et s'étendre de façon circonférentielle autour de ladite région centrale (3'), lesdites premières extrémités mutuellement adjacentes et lesdites deuxièmes extrémités mutuellement adjacentes, se composant d'un matériau flexible et fusible, et dans lequel ledit ensemble formant collier, une fois inter-ajusté, peut être placé en biais dans une position théoriquement fermée de sorte que lesdites premières extrémités des desdits éléments en forme de U se chevauchent, et les deuxièmes extrémités desdits éléments en forme de U se chevauchent, et dans lequel lesdites extrémités de chevauchement desdits éléments en forme de U sont adaptées pour fusionner entre elles autour de ladite région centrale (3') suite à l'application d'une énergie suffisante sur au moins une desdites parties de chevauchement dudit ensemble formant collier.

3. Dispositif selon la revendication 1, dans lequel les éléments allongés comprennent des sutures chirurgicales (13) composées d'un matériau fusible, dans lequel les sutures sont adaptées pour fusionner avec au moins une extrémité de collier (1) et une autre sous la forme d'une soudure sans noeud suite à l'application d'une énergie suffisante sur le collier.

4. Dispositif selon la revendication 2, dans lequel les éléments allongés comprennent des sutures chirurgicales (13) composées d'un matériau fusible, dans lequel les sutures sont adaptées pour fusionner avec au moins un ensemble formant collier (1') et un autre sous la forme d'une soudure sans noeud suite à l'application d'une énergie suffisante sur l'ensemble formant collier.

5. Dispositif selon la revendication 3, dans lequel le collier comprend un ou plusieurs directeurs d'énergie s'étendant à partir d'une surface faisant face vers l'intérieur du collier dans la région centrale, dans lequel les directeurs d'énergie définissent des régions de fusion des sutures et du collier et sont adaptés pour concentrer l'énergie appliquée au collier sur les régions de fusion de sorte qu'un ou plusieurs des éléments allongés et le collier puissent fusionner ensemble au niveau des régions de fusion.

6. Dispositif selon la revendication 4, dans lequel un élément en forme de U ou les deux incluent un ou plusieurs directeurs d'énergie s'étendant à partir d'une surface faisant face vers l'intérieur dudit un ou desdits deux éléments en forme de U dans la région centrale, dans lequel les directeurs d'énergie définissent des régions de fusion des sutures et de l'ensemble formant collier et sont adaptés pour concentrer l'énergie appliquée à l'ensemble formant collier sur les régions de fusion de sorte qu'un ou plusieurs des éléments allongés et l'ensemble formant collier puissent fusionner ensemble au niveau des régions de fusion.

7. Dispositif selon l'une quelconque des revendications 1 ou 3, dans lequel le collier comprend un ou plusieurs directeurs d'énergie situés sur une ou plusieurs surfaces opposées des extrémités dans la zone du chevauchement, dans lequel les directeurs d'énergie définissent des régions de fusion pour les extrémités dans la zone de chevauchement et sont adaptés pour concentrer l'énergie appliquée au collier sur les régions de fusion de sorte que les extrémités puissent fusionner ensemble.

8. Dispositif selon la revendication 2 ou 4, dans lequel un élément en forme de U ou les deux incluent un ou plusieurs directeurs d'énergie situés sur une ou plusieurs surfaces opposées des extrémités de ceux-ci dans la zone de chevauchement, dans lequel les directeurs d'énergie définissent des régions de fusion pour les extrémités dans des zones de chevauchement et sont adaptés pour concentrer l'énergie appliquée à l'ensemble formant collier sur les régions de fusion de sorte que les extrémités puissent fusionner ensemble.

9. Dispositif selon l'une quelconque des revendications précédentes, dans lequel les éléments allongés comportent une bande de matériau adaptée pour attacher une pluralité de structures anatomiques ensemble.

10. Dispositif selon l'une quelconque des revendications 1, 3, 5 ou 7, dans lequel le collier comprend une ou plusieurs saillies s'étendant à partir d'une surface faisant face vers l'intérieur du collier dans la région centrale et qui sont adaptées pour fixer ensemble les éléments allongés dans la région centrale.

11. Dispositif selon l'une quelconque des revendications 2, 4, 6 ou 8, dans lequel l'ensemble formant collier comprend une ou plusieurs saillies s'étendant à partir d'une surface faisant face vers l'intérieur d'un élément en forme de U ou des deux dans la région centrale quand elles sont inter-ajustées et qui sont adaptées pour fixer ensemble les éléments allongés dans la région centrale.

12. Dispositif selon les revendications 1 ou 2, dans lequel les éléments allongés comprennent des structures tissulaires vivantes.

13. Dispositif selon l'une quelconque des revendications précédentes, dans lequel l'énergie appliquée au collier ou à l'ensemble formant collier est de l'énergie ultrasonore.

14. Dispositif selon l'une quelconque des revendications précédentes, dans lequel l'énergie appliquée au collier ou à l'ensemble formant collier est choisie dans le groupe comprenant de l'énergie thermique, de l'énergie optique, de l'énergie électrique et des sources de courant.

15. Dispositif selon l'une quelconque des revendications précédentes, dans lequel le collier se compose d'un matériau polymère thermoplastique.

16. Kit pour fixer ensemble une ou plusieurs structures tissulaires vivantes allongées, le kit comprenant :
un dispositif selon la revendication 1, dans lequel les un ou plusieurs éléments allongés sont des structures tissulaires vivantes ; et
un outil de fusion (20) servant à comprimer le collier autour des structures tissulaires de sorte que les structures de tissu soient solidement retenues dans la région centrale, et servant à appliquer de l'énergie au collier pour fusionner ensemble les parties se chevauchant, dans lequel l'outil de fusion comprend une source d'énergie, une tête de soudage (WH) pour effectuer la fusion, et un terminal effecteur (24) pour soutenir le collier pendant la compression et la fusion.

17. Kit selon la revendication 16, dans lequel la source d'énergie est un capteur ultrasonore, et l'outil de fusion comprend en outre une corne d'air s'étendant entre le capteur ultrasonore et la tête de soudage pour transmettre l'énergie ultrasonore du capteur à la tête de soudage.

18. Kit selon la revendication 16 ou 17, dans lequel le terminal effecteur comprend un ou plusieurs éléments de mâchoire adaptés pour un déploiement sélectif autour d'au moins une partie du collier pour former une enclume de soudage par ultrasons.

19. Kit pour fixer ensemble une pluralité de sutures chirurgicales ensemble sans noeud, le kit comprenant :
un dispositif selon la revendication 1, dans lequel les un ou plusieurs éléments allongés sont des sutures chirurgicales ; et
un outil de fusion (20) servant à comprimer le collier autour des sutures chirurgicales de sorte que les sutures chirurgicales soient solidement retenues dans la région centrale, et servant à appliquer de l'énergie au collier pour fusionner ensemble les parties se chevauchant, dans lequel l'outil de fusion comprend une source d'énergie, une tête de soudage (WH) pour effectuer la fusion, et un terminal effecteur (24) pour soutenir le collier pendant la compression et la fusion.

20. Kit selon la revendication 19, dans lequel la source d'énergie est un transducteur ultrasonore, et l'outil de fusion comprend en outre une corne d'air s'étendant entre le capteur ultrasonore et la tête de soudage pour transmettre l'énergie ultrasonore du transducteur à la tête de soudage, et dans lequel le terminal effecteur comprend un ou plusieurs éléments de mâchoire adaptés pour un déploiement sélectif autour d'au moins une partie du collier pour former une enclume de soudage par ultrasons.

21. Kit selon l'une quelconque des revendications 16, 17, 19 ou 20, dans lequel le terminal effecteur comprend une paire de mâchoires opposées qui sont mobiles l'une par rapport à l'autre.

22. Kit selon la revendication 18, dans lequel les éléments de mâchoire sont opposés et peuvent être déplacés l'un par rapport à l'autre.

23. Kit selon l'une quelconque des revendications 16, 17, 19 ou 20, dans lequel le terminal effecteur comprend une paire de mâchoires qui sont placées en biais de façon élastique l'une envers l'autre.

24. Kit selon la revendication 18, dans lequel les éléments de mâchoire sont placés en biais de façon élastique l'un envers l'autre.
